# EUROPEAN PATENT APPLICATION

(11) **EP 3 326 530 A1**
(43) Date of publication of application: **30.05.2018**
(21) Application number: 17203241.9
(22) Date of filing: 23.11.2017
(51) Int. Cl.: A61B 5/1455, A61B 5/00, A61B 5/0476

(54) **SENSOR FOR DEPTH OF ANESTHESIA AND CEREBRAL BLOOD OXYGEN SATURATION**

(30) Priority: 28.11.2016 KR 20160158908
(71) Applicant: Charm Engineering Co., Ltd., Gyeonggi-do 17118 (KR)
(72) Inventor: KIM, Kwang Moo, 13525 Seongnam-si (KR); HONG, Seung Kyun, 16084 Uiwang-si (KR); CHOI, Sang Woo, 14231 Gwangmyeong-si (KR); LEE, Kyu Hong, 17046 Yongin-si, (KR)
(74) Representative: Wablat Lange Karthaus

(57) **Abstract**

Provided is an integrated depth-of-anesthesia/cerebral-oxygen-saturation detection sensor. Particularly, the
depth-of-anesthesia/cerebral-oxygen-saturation detection sensor includes a strap and a sensing unit that is arranged on the strap, and sensing unit includes a depth-of-anesthesia sensor and a cerebral oxygen saturation sensor that are arranged on the same plane. Therefore, it is possible to simultaneously measure a depth of anesthesia and cerebral oxygen saturation with a single detection sensor, so that it is possible to reduce cost.

## Description

### [Technical Field]

The present invention relates to an integrated depth-of-anesthesia/cerebral oxygen saturation detection sensor, and more particularly, to an integrated depth-of-anesthesia/cerebral oxygen saturation detection sensor capable of measuring a depth of anesthesia and cerebral oxygen saturation with a single sensor.

### [Background Art]

Generally, if a subject (patient) suffers from pain during medical care such as surgery and treatment or skin care, the pain is eliminated or reduced by blocking neurotransmission through anesthetics.

In particular, If surgery is performed on a patient having a sever disease or symptom, the patient needs to be put under general anesthetics to remove all of the body reactions. In addition, the patient's anesthetized state needs to be continuously monitored under such general anesthetics.// In other words, the patient's anesthetized state needs to be checked by detecting the depth of anesthesia, and the operation needs to be performed in a sufficiently anesthetized state so as to prevent a problem in that the patient experiences psychological distress due to awakening during surgery. Currently, the detection of the depth of anesthesia is performed by attaching an EEG sensor to a human body and then collecting and amplifying biological signals including high and low potential differences.

On the other hand, cerebral oxygen saturation indicating oxygen saturation of cortical tissues is used as an important parameter in clinical field such as hypoxia, neonatal monitoring, or emergency medicine and, particularly, the cerebral oxygen saturation is very important in clinical management of critically ill patients. Currently, the detection of the cerebral oxygen saturation is performed by attaching a sensor to a human body and then emitting red light and near-infrared light to detect a degree of oxidation of hemoglobin.

Both of the depth-of-anesthesia sensor or the cerebral oxygen saturation sensor are important means to effectively monitor an awakened level of a patient. Since the two sensors are manufactured as separate products and are individually used, the sensors are cumbersome in terms of usage, and it is difficult to integrally monitor the patient's awakened level.

### [Cited Literature]

### [Patent Document]

Korean Patent Application No. 10-2010-0035977 (Registration No. 10-1079785, titled, "Electroencephalogram Analyzer for Calculating Depth of Anesthesia Index)

### [Disclosure]

### [Technical Problem]

The present invention is conceived to solve the above-described problems in the related art, and an object of the present invention is to an integrated depth-of-anesthesia/cerebral-oxygen-saturation detection sensor where a depth-of-anesthesia sensor and a cerebral oxygen saturation sensor are installed on a single strap, so that a depth of anesthesia and cerebral oxygen saturation can be measured by the single sensor at the same time.

### [Technical Solution]

In order to achieve the above object, according to an aspect of the present invention, there is provided an integrated depth-of-anesthesia/cerebral-oxygen-saturation detection sensor including a strap and a sensing unit arranged on the strap, wherein the sensing unit includes a depth-of-anesthesia sensor and a cerebral oxygen saturation sensor which are arranged on the same plane.

Herein, the depth-of-anesthesia sensor may include a ground electrode; and a reference electrode that is arranged to be separated from the ground electrode; and first and second measurement electrodes that are arranged on two sides of the ground electrode and the reference electrode so that the ground electrode and the reference electrode are located between the ground electrode and the reference electrode,

In addition, the cerebral oxygen saturation sensor may include: a pair of light sources that irradiate a head with red light and near-infrared light having different wavelengths; a pair of first light detection units that receives light reflected from head skin or skull; and a pair of second light detection unit that receives light reflected from brain.

In addition, the pair of light sources may be located between the first measurement electrode and the reference electrode and between the second measurement electrode and the ground electrode, and the pair of first light detection units may be arranged between the light source and the reference electrode and between the light source and the ground electrode, and the pair of second light detection unit may be located to be separated from each other between the reference electrode and the ground electrode.

In addition, the strap may be formed to have wrinkles in a portion where the depth-of-anesthesia sensor and the cerebral oxygen saturation sensor are not arranged.

### [Advantageous Effects]

In an integrated depth-of-anesthesia/cerebral oxygen saturation detection sensor having the above-described configuration according to the present invention, a depth-of-anesthesia sensor and a cerebral oxygen saturation sensor are provided on a single strap, and thus, since a depth of anesthesia and cerebral oxygen saturation can be measured at the same time, the detection sensor can be conveniently used, and since there is no need to separately manufacture detection sensors for measuring the depth of anesthesia and the cerebral oxygen saturation, it is possible to advantageously reduce cost.

### [Description of Drawings]

Fig. 1 is a perspective view of an integrated depth-of-anesthesia/cerebral-oxygen-saturation detection sensor according to the present invention.
Fig. 2 is a view illustrating a use state of the integrated depth-of-anesthesia/cerebral-oxygen-saturation detection sensor according to the present invention.

### [Best Mode]

Hereinafter, embodiments of an integrated depth-of-anesthesia/cerebral-oxygen-saturation detection sensor according to the present invention will be described in detail with reference to the accompanying drawings.

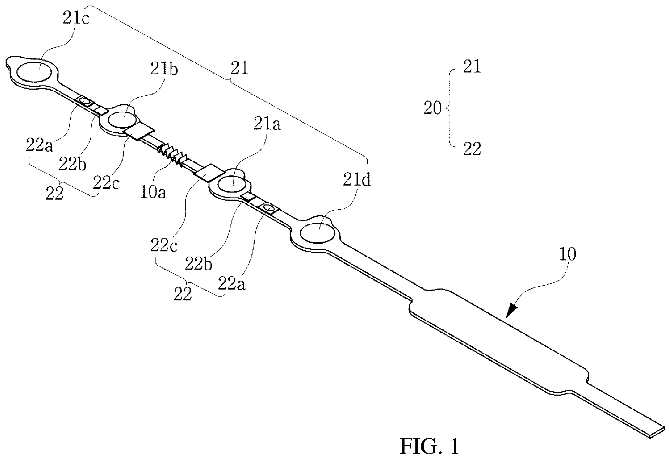

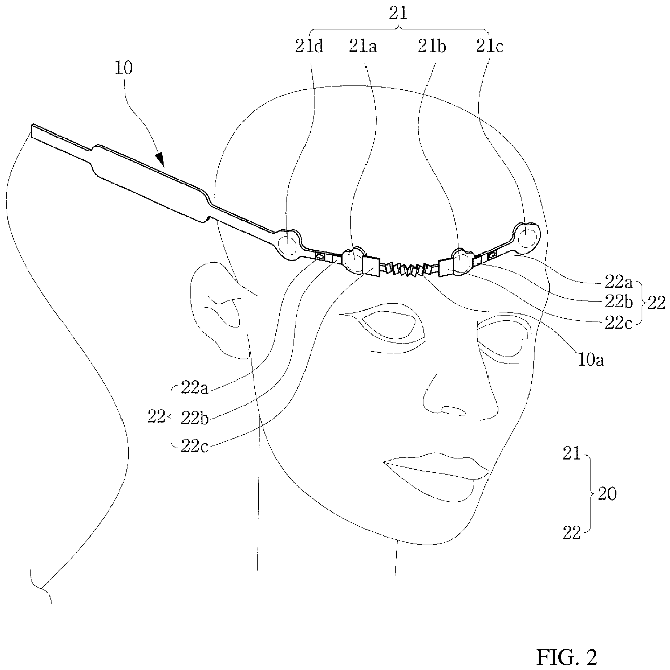

Fig. 1 is a perspective view illustrating an integrated depth-of-anesthesia/cerebral-oxygen-saturation detection sensor according to the present invention, and Fig. 2 is a view illustrating a use state of the integrated depth-of-anesthesia/cerebral-oxygen-saturation detection sensor according to the present invention.

An integrated
depth-of-anesthesia/cerebral-oxygen-saturation detection sensor according to the present invention is configured to include a strap 10 and a sensing unit 20 arranged on the strap 10.

A material of the strap 10 needs to be selected in consideration of characteristics of a human body, particularly, the head of the human body to which the integrated depth-of-anesthesia/cerebral-oxygen-saturation detection sensor according to the present invention are attached.

Namely, in consideration of the fact that the surface of the head is not flat but curved, a material that is flexible so as to be easily bended and folded needs be selected, a material that is highly adhesive to the human body so as to be well adhered to the forehead or scalp and to be well detached needs to be selected, and a material that does not irritate the human body even if the strap is frequently attached and detached needs to be selected to manufacture the strap. Particularly, the above-mentioned effect can be obtained by using a hydrogel which is capable of adhering to the human body several times and, at the same time, not causing irritation to the skin.

The sensing unit 20 is configured to include a depth-of-anesthesia sensor 21 and a cerebral oxygen saturation sensor 22. The depth-of-anesthesia sensor 21 and the cerebral oxygen saturation sensor 22 are arranged on the same plane.

The depth-of-anesthesia sensor 21 is configured to include a ground electrode 21a, a reference electrode 21b, and first and second measurement electrodes 21c and 21d that are arranged on two sides of the ground electrode 21a and the reference electrode 21b so as to be located between the ground electrode 21a and the reference electrode 21b.

The ground electrode 21a is in contact with a portion of the human body for electrical safety of the subject during the measurement of the biological signal, so that the subject is maintained to be in the grounded state. In other words, the ground electrode 21a prevents a safety accident caused by an abnormal voltage during the measurement of the biological signal, and when a fault current occurs, the ground electrode performs a function of discharging the current quickly to the outside of the human body to suppress an increase in an abnormal potential and ensuring the electrical safety of the subject. The ground electrode 21a is provided on one side of the strap 10.

The reference electrode 21b is an electrode as a reference for measuring a biological signal and is provided as a reference for measuring a potential difference between the first and second measurement electrodes 21c and 21d. The reference electrode 21b is provided on one side of the strap 10 and is separated from the ground electrode 21a by a predetermined distance.

The first measurement electrode 21c is attached to the head of the human body and senses a biological signal in the anesthetized state or during the anesthetic operation to measure and detect brain waves generated in the head. The first measurement electrode 21c is arranged at one of the two sides of the reference electrode 21b and is arranged at a position separated from the ground electrode 21a.
Similarly to the first measurement electrode 21c, the second measurement electrode 21d is attached to the head of the human body and senses a biological signal in the anesthetized state or during the anesthetic operation to measure and detect brain waves generated in the head.//
Since the measurement electrodes performs the function of measuring and detecting the biological signal, collecting and analyzing the biological signal in a wider range is more advantageous in consideration of the precision and efficiency of the measurement. Particularly, in the case where the brain waves are measured by the measurement electrodes like the present invention, it is preferable that the brain waves of the left brain and right brain are measured. Therefore, when the first measurement electrode 21c measures the brain waves of the left brain, it is preferable that the second measurement electrode 21d measures the brain waves.

Accordingly, the second measurement electrode 21d is arranged so as to be separated from the first measurement electrode 21c as far as possible. That is, the secondmeasurement electrode 21d is arranged on one of the two side of the ground electrode 21a and is arranged at a position separated from the first measurement electrode 21c.

The cerebral oxygen saturation sensor 22 irradiates the red light and the near-infrared light, calculates the ratio of the reflected red light and infrared light components, and then estimates the oxygen saturation based on the calculated ratio of the red light and infrared light components. The cerebral oxygen saturation sensor is configured to include a pair of first light detection units 22b and a pair of second light detection units 22c.

The light source 22a irradiates the head with red light and near-infrared light having different wavelengths. One light source 22a is arranged between the first measurement electrode 21c and the reference electrode 21b and the other is arranged between the second measurement electrode 21d and the ground electrode 21a.

The first light detection unit 22b receives the red light and the near-infrared light which are emitted from the light source 22a and are reflected from the head skin or the skull. That is, the first light detection unit 22b receives the light that cannot penetrate into a deep portion of the head and is reflected at a shallow portion of the head such as a head skin or a skull. One of the first light detection unit 22b is arranged between the light source 22a and the reference electrode 21b and the other is arranged between the light source and the ground electrode 21a.

In the case where the red light and the near-infrared light emitted from the light source 22a are reflected from the brain, the second light detection unit 22c receives the light. That is, the second light detection unit 22c receives the light that passes through a shallow portion such as the head skin or a skull, reaches the brain, and is reflected from the brain. Each of a pair of the second light detection units 22c is arranged between the reference electrode 21b and the ground electrode 21a so that the second light detection units 22c are separated from each other.

Since the light sources 22a, first light detection units 22b and the second light detection units 22c are located in the above-described manner, one of the light sources 22a, one of the first light detection units 22b, and one of the second light detection units 22c are arranged in the vicinity of the reference electrode 21b, and the other of the light sources 22a and the other of the first light detection units 22b, and the other of the second light detection units 22c are arranged in the vicinity of the ground electrode 21a. Therefore, when one of the light sources 22a, one of the first light detection units 22b, and one of the second light detection units 22c arranged in the vicinity of the reference electrode 21b measure the oxygen saturation of the left brain, the other of the light sources 22a and the other of the first light detection units 22b, and the other of the second light detection units 22c arranged in the vicinity of the ground electrode 21a measure the oxygen saturation of the right brain.

Meanwhile, the strap 10 may be formed to have wrinkles 10a in a portion where the depth-of-anesthesia sensor 21 and the cerebral oxygen saturation sensor 22 are not arranged. As described above, since the surface of the head of a person is not flat but curved, it is also important to select a flexible material for the strap 10 itself in order to effectively adhere the strap 10 to such a head shape. In addition, it is also necessary to form the strap 10 to have an auxiliary means such as wrinkles 10a so that the flexible strap 10 can be well bended.

In addition, if the width of the portion of the strap 10 where the depth-of-anesthesia sensor 21 and the cerebral oxygen saturation sensor 22 are not arranged is smaller than that of the portion where the depth-of-anesthesia sensor 21 and the cerebral oxygen saturation sensor 22 are arranged, the strap 10 may be well bended. Therefore, the depth-of-anesthesia sensor 21 and the cerebral oxygen saturation sensor 22 can be attached better.

### [Reference Numerals]

10: strap
10a: wrinkle
20: sensing unit
21: depth-of-anesthesia sensor
21a: ground electrode
21b: reference electrode
21c: first measurement electrode
21d: second measurement electrode
22: cerebral oxygen saturation sensor
22a: light source
22b: first light detection unit
22c: second light detection unit

## Claims

1. An integrated depth-of-anesthesia/cerebral-oxygen-saturation detection sensor comprising:
a strap (10); and
a sensing unit (20) arranged on the strap (10),
wherein the sensing unit (20) includes a depth-of-anesthesia sensor (21) and a cerebral oxygen saturation sensor (22) that are arranged on the same plane.

2. The integrated depth-of-anesthesia/cerebral-oxygen-saturation detection sensor according to claim 1, wherein the depth-of-anesthesia sensor (21) includes:
a ground electrode (21a);
a reference electrode (21b) that is arranged to be separated from the ground electrode (21a); and
first and second measurement electrodes (21c and 21d) that are arranged on two sides of the ground electrode (21a) and the reference electrode (21b) so that the ground electrode (21a) and the reference electrode (21b) are located between the ground electrode (21a) and the reference electrode (21b).

3. The integrated depth-of-anesthesia/cerebral-oxygen-saturation detection sensor according to claim 2, wherein the cerebral oxygen saturation sensor (22) includes:
a pair of light sources (22a) that irradiate a head with red light and near-infrared light having different wavelengths;
a pair of first light detection units (22b) that receive light reflected from head skin or skull; and
a pair of second light detection units (22c) that receive light reflected from brain.

4. The integrated depth-of-anesthesia/cerebral-oxygen-saturation detection sensor according to claim 3,
wherein the pair of light sources (22a) are located between the first measurement electrode (21c) and the reference electrode (21b) and between the second measurement electrode (21d) and the ground electrode (21a),
wherein the pair of first light detection units (22b) are located between the light source (22a) and the reference electrode (21b) and between the light source (22a) and the ground electrode (21a), and
wherein the pair of second light detection units (22c) are located to be separated from each other between the reference electrode (21b) and the ground electrode (21a).

5. The integrated depth-of-anesthesia/cerebral-oxygen-saturation detection sensor according to claim 1,
wherein the strap (10) is formed to have wrinkles (10a) in a portion where the depth-of-anesthesia sensor (21) and the cerebral oxygen saturation sensor (22) are not arranged.
